# EUROPÄISCHE PATENTSCHRIFT

(11) **EP 2 680 713 B1**
(45) Veröffentlichungstag und Bekanntmachung des Hinweises auf die Patenterteilung: **20.12.2017**
(21) Anmeldenummer: 12710460.2
(22) Anmeldetag: 29.02.2012
(51) Int. Cl.: A23L 33/18, A23L 33/125, A23L 33/15, A23L 33/16, A23L 33/22, A61K 38/39, A61K 38/01, A61P 19/10

(54) **ZUSAMMENSETZUNG FÜR ERNÄHRUNGSZWECKE**
COMPOSITION FOR NUTRITION PURPOSES
COMPOSITION À USAGE NUTRITIONNEL

(30) Priorität: 01.03.2011 DE 102011000997
(43) Veröffentlichungstag der Anmeldung: 08.01.2014
(73) Patentinhaber: GELITA AG, 69412 Eberbach (DE)
(72) Erfinder: OESSER, Steffen, 24960 Glücksburg (DE); SCHOTT, Annelore, 69115 Heidelberg (DE); HAUSMANNS, Stephan, 69126 Heidelberg (DE)
(74) Vertreter: Hoeger, Stellrecht & Partner Patentanwälte mbB
(86) Internationale Anmeldenummer: PCT/EP2012/053413
(87) Internationale Veröffentlichungsnummer: WO 2012/117012

(56) Entgegenhaltungen:
- EP-A1- 0 596 546
- EP-A1- 2 233 006
- EP-B1- 0 777 491
- WO-A1-98/31345
- WO-A1-2004/014152
- WO-A1-2007/031269
- CN-A- 101 496 579
- CN-B- 101 496 576
- DE-C1- 4 320 816
- JP-A- 2008 247 809
- US-A1- 2007 237 874
- US-A1- 2009 311 409
- US-A1- 2011 039 767

## Beschreibung

Die vorliegende Erfindung betrifft eine Zusammensetzung für Ernährungszwecke, insbesondere zur Erhaltung und/oder Verbesserung der Gesundheit der Knochen.

Die Knochen der Wirbeltiere und somit auch die des Menschen verdanken ihre hohe Festigkeit und mechanische Stabilität dem besonderen Aufbau der Knochenmatrix, die von den Osteoblasten synthetisiert wird. Die beiden wesentlichen Komponenten der Knochenmatrix sind zum Einen ein Gerüst aus quervernetztem Kollagen, wobei es sich speziell im Fall der Knochenmatrix um Kollagen Typ I handelt. Die Quervernetzung erfolgt hierbei über die Aminosäuren Lysin und Hydroxylysin. Bei der zweiten Komponente handelt es sich um Hydroxylapatit (auch als Apatit-(CaOH) bezeichnet), welches in die Knochenmatrix eingelagert wird (Mineralisation der Knochen). Dieser Aufbau des Knochens ist grob vergleichbar mit demjenigen von Stahlbeton, wo sich die Eigenschaften des Stahlgerüsts (entsprechend dem Kollagen) und des Betons (entsprechend dem Hydroxylapatit) ebenfalls unter Ausbildung einer äußerst tragfähigen Struktur ergänzen.

Im Gegensatz zu vielen anderen Gewebetypen weist der Knochen eine hohe Regenerationsfähigkeit auf, d.h. die extrazelluläre Knochenmatrix wird kontinuierlich auf- und wieder abgebaut. Bei einer Störung dieses Gleichgewichts, also einem unzureichenden Aufbau von neuer Knochenmatrix, kommt es zu einem Knochenschwund. Diese Abnahme der Knochendichte wird als Osteoporose bezeichnet und kann verschiedene Ursachen haben. Typischerweise tritt die Osteoporose mit zunehmendem Lebensalter auf (meist ab Beginn des fünften Lebensjahrzehnts), besonders häufig bei Frauen nach der Menopause (postmenopausale Osteoporose).

Es ist bekannt, dass durch die orale Verabreichung von Kollagenhydrolysat, d.h. durch enzymatische Hydrolyse hergestellter Kollagenpeptide, dem Verlust der Knochendichte bei Osteoporose entgegengewirkt werden kann. Die EP 0 777 491 B1 offenbart z.B. die Verwendung von Kollagenhydrolysat mit einem mittleren Molekulargewicht von 1 bis 40 kDa zur Behandlung von postmenopausaler Osteoporose. Es ist anzunehmen, dass dieser vorteilhafte Effekt von Kollagenhydrolysat auf die Gesundheit der Knochen auf einer Stimulation der Kollagenbiosynthese durch die Osteoblasten beruht, ähnlich wie dies für Chondrozyten bereits *in vitro* nachgewiesen werden konnte (siehe S. Oesser und J. Seiffert (2003), Cell Tissue Research (311) 393-399). JP 2008 247809 beschreibt Kollagenhydrolysat mit 1000-4000 Dalton für die Verwendung zur Vorbeugung und/oder Behandlung von Osteoporose. CN 101 496 576 offenbart Kollagenhydrolysat mit Molekulargewicht unterhalb von 1000 Dalton und Präbiotika für die Verwendung zur Knochenbruch. Der Erfindung liegt die Aufgabe zugrunde, eine Zusammensetzung für Ernährungszwecke mit einer verbesserten Wirksamkeit in Bezug auf den Aufbau der Knochenmatrix vorzuschlagen.

Diese Aufgabe wird erfindungsgemäß dadurch gelöst, dass die Zusammensetzung 20 bis 80 Gew.% Kollagenhydrolysat mit einem mittleren Molekulargewicht im bereich von 1500 bis 8000 DA und 20 bis 80 Gew.% eines oder mehrerer Präbiotika umfasst, jeweils bezogen auf die Trockenmasse der Zusammensetzung. Die Erfindung wird am besten durch die Ansprüche beschirieben. Der erfindungsgemäßen Kombination von Kollagenhydrolysat mit einem oder mehreren Präbiotika beruht dabei auf der Überlegung, dass für eine effektive Regeneration der Knochenmatrix nicht nur die Biosynthese von Kollagen, sondern auch die Bildung und Einlagerung von Hydroxylapatit in ausreichender Menge ermöglicht werden muss. Der limitierende Faktor hierbei ist die Versorgung der Knochenmatrix mit Calcium, wobei nicht die ausreichende Aufnahme von Calcium mit der Nahrung das Problem darstellt (diese ist bei einer ausgewogenen Ernährung in der Regel gewährleistet), sondern die ausreichende Resorption des aufgenommenen Calciums im Darm, die insbesondere aufgrund der Bildung von schwer löslichen Calciumsalzen limitiert ist.

Es hat sich nun gezeigt, dass die Resorption von Calcium und damit die Mineralisation der Knochen durch die Verabreichung von Präbiotika verbessert werden kann. Bei Präbiotika handelt es sich allgemein um nicht verdaubare Lebensmittelbestandteile, die das Wachstum und/oder die Aktivität bestimmter

Mikroorganismen der Darmflora gezielt anregen und dadurch einen positiven Einfluss auf die Gesundheit aufweisen. Der genaue Mechanismus, über den sich dieser Effekt der Präbiotika vorteilhaft auf die Calciumresorption auswirkt, ist zwar noch nicht ganz geklärt, der oben genannte positive Effekt wurde aber z.B. für die Verabreichung von Fruktanen an Kinder im Alter von ca. 12 Jahren belegt (siehe S. Abrams et al. (2005), American Journal of Clinical Nutrition (82) 471-476).

Die gemeinsame Verabreichung von Kollagenhydrolysat und Präbiotika, um die Bildung der beiden wesentlichen Bestandteile der Knochenmatrix zu stimulieren und dadurch einen synergetischen Effekt in Bezug auf die Gesundheit der Knochen zu erzielen, wurde im Stand der Technik bisher nicht beschrieben oder in Erwägung gezogen, insbesondere auch nicht zum Zweck der Vorbeugung und/oder Behandlung von Osteoporose.

Der Nutzen der erfindungsgemäßen Zusammensetzung ist dabei aber nicht auf diese spezifische Wirkung beschränkt, da sowohl Präbiotika als auch Kollagenhydrolysat bekanntermaßen weitere vorteilhafte Eigenschaften unter gesundheitlichen Aspekten aufweisen. Insbesondere hat die orale Verabreichung von Kollagenhydrolysat einen vorteilhaften Effekt auf das Knorpelgewebe und kann dadurch degenerativen Gelenkerkrankungen vorbeugen (siehe A. Bello und S. Oesser (2006), Currant Medical Research and Opinion (22) 2221-2232).

Als besonders vorteilhaft ist auch hervorzuheben, dass es sich bei den beiden Bestandteilen der erfindungsgemäßen Zusammensetzung rechtlich um Lebensmittel handelt, bei denen selbst im Fall einer Überdosierung keine nachteiligen Wirkungen bekannt sind. Die Zusammensetzung benötigt aus diesem Grund keinerlei arzneimittelrechtliche oder sonstige Zulassung, sondern kann als Zusammensetzung für Ernährungszwecke in Form eines Nahrungsergänzungsmittels oder auch als Bestandteil von Lebensmitteln angeboten werden.

Die Zusammensetzung muss gemäß der Erfindung 20 bis 80 Gew.% Kollagenhydrolysat mit einem mittleren Molekulargewicht im bereich von 1500 bis 8000 DA und 20 bis 80 Gew.% des oder der Präbiotika umfassen, wobei die Summe aus Kollagenhydrolysat und Präbiotika den wesentlichen Anteil der Zusammensetzung (also mehr als 50 Gew.%) ausmachen sollte. Um eine ausreichende Versorgung mit beiden Bestandteilen zu gewährleisten, ist es bevorzugt, wenn der Anteil des Kollagenhydrolysats und des oder der Präbiotika jeweils 40 bis 60 Gew % jeweils bezogen auf die Trockenmasse der Zusammensetzung. Die Zusammensetzung kann insbesondere eine Mischung der beiden Bestandteile im Gewichtsverhältnis von etwa 1:1 umfassen.

Das Kollagenhydrolysat, das im Rahmen der vorliegenden Erfindung zum Einsatz kommt, ist bevorzugt durch enzymatische Hydrolyse von Kollagen aus tierischem Bindegewebe hergestellt, insbesondere von Schweinen, Rindern, Geflügel oder Fischen. Als bevorzugte Ausgangsmaterialien werden häufig Schweineschwarten oder Rinderspalt eingesetzt. Das Kollagenhydrolysat kann aber auch durch Hydrolyse von Kollagen aus den Knochen verschiedener Tierarten gewonnen werden, insbesondere aus Ossein. Verfahren zur Herstellung entsprechender Hydrolysate sind aus dem Stand der Technik bekannt. Günstigerweise weist das Kollagenhydrolysat ein mittleres Molekulargewicht im Bereich von 1500 bis 8000 Da auf. Die Molekulargewichtsverteilung von Kollagenhydrolysaten kann insbesondere durch Gelpermeationschromatografie unter Verwendung von definierten Kollagenpeptiden als Eichpeptide bestimmt werden. Bevorzugt liegt das mittlere Molekulargewicht im Bereich von 2500 bis 3500 Da. Insbesondere für Hydrolysate in diesem Molekulargewichtsbereich liegen umfangreiche Daten zur Stimulierung der Biosynthese von extrazellulären Matrixproteinen vor.

Das oder die Präbiotika, die in der erfindungsgemäßen Zusammensetzung eingesetzt werden, sind bevorzugt ausgewählt aus Oligo- und/oder Polysacchariden. Oligo- und Polysaccharide machen den Großteil der bekannten präbiotisch wirksamen Substanzen aus, wobei sich durch die Verwendung derartiger Substanzen der zusätzliche vorteilhafte Effekt ergibt, dass der Geschmack der erfindungsgemäßen Zusammensetzung im Vergleich zu reinem Kollagenhydrolysat sehr deutlich verbessert wird. Obwohl mittels entsprechender Verfahren Kollagenhydrolysate hergestellt werden können, die üblicherweise als geschmacksneutral bezeichnet werden, nehmen viele Verbraucher eine als "leimig" bezeichnete geschmackliche Beeinträchtigung wahr. Überraschenderweise können die negativen geschmacklichen Komponenten von Kollagenhydrolysat durch die Kombination mit präbiotischen Oligo- und/oder Polysacchariden fast vollständig eliminiert werden.

Das oder die Präbiotika sind bevorzugt ausgewählt aus Inulin, Fruktanen, Galacto-Oligosacchariden (GOS), Fructo-Oligosacchariden (FOS), resistenten Maltodextrinen, Polydextrose und Mischungen hiervon. Diese umfassen sowohl natürlich vorkommende als auch synthetisch hergestellte Saccharide. Bei Inulin handelt es sich um ein Fruktan, welches bis zu 100 Fructose-Einheiten umfasst sowie eine endständige Glucose-Einheit. Fructo-Oligosaccharide und Galacto-Oligosaccharide umfassen ausschließlich Fructose- bzw. Galactose-Einheiten (in der Regel bis zu 10), und bei Polydextrose handelt es sich um ein synthetisches Polysaccharid aus Glucose-, Sorbit- und Zitronensäure-Einheiten.

Bei einer bevorzugten Ausführungsform der Erfindung umfasst die Zusammensetzung als weiteren Bestandteil mindestens ein lösliches Calciumsalz. Hierdurch wird gleichzeitig eine ausreichende Versorgung des Anwenders mit Calcium, unabhängig von dessen sonstigen Ernährungsgewohnheiten, sichergestellt.

Es kommen prinzipiell alle löslichen Calciumsalze in Frage, die nicht toxisch sind oder sonstige nachteilige Wirkungen haben. Vorzugsweise ist das mindestens eine lösliche Calciumsalz ausgewählt aus Calciumcitrat, Calciumlactat, Calciumgluconat, Calciumlactatgluconat, Calciumlactobionat und Mischungen hiervon.

Die Menge des mindestens einen Calciumsalzes wird günstigerweise so gewählt, dass der Anteil an Calcium ca. 0,1 bis ca. 10 Gew.% beträgt, bezogen auf die Trockenmasse der Zusammensetzung. Weiter bevorzugt beträgt der Anteil an Calcium ca. 0,5 bis ca. 5 Gew.%.

Die erfindungsgemäße Zusammensetzung kann darüber hinaus noch weitere Bestandteile enthalten, die die Gesundheit der Knochen fördern (insbesondere Vitamin D und/oder Vitamin K) oder die allgemein eine gesundheitlich vorteilhafte Wirkung haben (z.B. weitere Mineralstoffe wie Magnesium, Vitamin C und/oder Glucosamin).

Des Weiteren kann die erfindungsgemäße Zusammensetzung weitere Zusatz- oder Hilfsstoffe in untergeordneten Mengenanteilen enthalten, wie z.B. Süßstoffe, Aromastoffe, Farbstoffe und/oder Trennmittel.

Gemäß einer bevorzugten Ausführungsform der Erfindung liegt die Zusammensetzung in Form eines Pulvers oder eines Granulats vor. In dieser Form ist die Zusammensetzung gut lagerfähig und kann vom Anwender nach Belieben dosiert und z.B. in Wasser oder einem Getränk aufgelöst werden. Das Kollagenhydrolysat und das oder die Präbiotika sowie ggf. weitere Bestandteile können nach dem Mischen gemeinsam vermahlen werden, um eine Zusammensetzung mit einer einheitliche Partikelgröße zu erhalten.

Die erfindungsgemäße Zusammensetzung kann aber auch in Form einer Lösung oder einer Suspension (je nach Löslichkeit des oder der Präbiotika) vorliegen. Das Anbieten der Zusammensetzung in einer solchen trinkfertigen, flüssigen Form (z.B. in entsprechend portionierten Ampullen) ermöglicht dem Verbraucher eine besonders einfache Anwendung.

Die erfindungsgemäße Zusammensetzung für Ernährungszwecke hat aufgrund ihrer Bestandteile eine allgemeine gesundheitsfördernde Wirkung. Ein wesentlicher Aspekt der vorliegenden Erfindung betrifft jedoch die Erhaltung und/oder Verbesserung der Gesundheit der Knochen, insbesondere die Vorbeugung und/ oder Behandlung von Osteoporose, durch das oben beschriebene Zusammenspiel von Kollagenhydrolysat (Stimulation der Kollagensynthese) und des oder der Präbiotika (Verbesserung der Calciumresorption und dadurch Stimulierung der Einlagerung von Hydroxylapatit in die Knochenmatrix).

Die erfindungsgemäße Zusammensetzung ist insbesondere geeignet zur Vorbeugung und/oder Behandlung von postmenopausaler Osteoporose. Des Weiteren ist die Verabreichung der erfindungsgemäßen Zusammensetzung auch vorteilhaft bei Patienten mit einer diagnostizierten Knochenmetastasenbildung.

Die erfindungsgemäße Zusammensetzung ist allgemein für Ernährungszwecke vorgesehen, was im Rahmen der Erfindung jede Form der Verabreichung an den Menschen oder auch an Tiere umfasst, sei es als Nahrungsmittel, Nahrungsergänzungsmittel oder pharmazeutisches Präparat.

Gemäß einer bevorzugten Ausführungsform der Erfindung wird die Zusammensetzung als Nahrungsergänzungsmittel verwendet, insbesondere in Form von Tabletten, Kapsel, Capsetten oder Sachets. Alternativ kann auch eine Lösung oder Suspension als Nahrungsergänzungsmittel angeboten werden (siehe oben). Dies ermöglicht es dem Anwender, eine vorgegebene bzw. definierbare Menge der erfindungsgemäßen Zusammensetzung zusätzlich zu seiner normalen Ernährung aufzunehmen.

Eine Tagesdosis, die auf eine oder mehrere Tabletten usw. aufgeteilt sein kann, enthält vorzugsweise ca. 3 bis ca. 15 g der erfindungsgemäßen Zusammensetzung, weiter bevorzugt ca. 5 bis ca. 10 g.

Im Rahmen der vorliegenden Erfindung kann die Zusammensetzung aber auch als Bestandteil von Lebensmitteln verwendet werden (so genanntes "functional food"), insbesondere als Bestandteil von Milchprodukten (z.B. Joghurt) oder von Süßwaren (z.B. Backwaren, Schokoladenriegel usw.). Im Zuge der Herstellung kann entweder die erfindungsgemäße Zusammensetzung als solche dem Lebensmittel zugegeben werden, oder deren einzelne Bestandteile, also Kollagenhydrolysat und Präbiotikum, ggf. auch zu verschiedenen Zeitpunkten des Herstellungsverfahrens.

Diese und weitere Vorteile der Erfindung werden anhand der folgenden Beispiele unter Bezugnahme auf die Figuren näher erläutert. Es zeigen im Einzelnen:
- Figur 1:: Ein Diagramm zur sensorischen Bewertung einer erfindungsgemäßen Zusammensetzung im Vergleich zu deren einzelnen Bestandteilen; und
- Figur 2:: ein Diagramm zur sensorischen Bewertung von verschiedenen erfindungsgemäßen Zusammensetzungen.

### Beispiel 1

Eine erfindungsgemäße Zusammensetzung für Ernährungszwecke wurde hergestellt, indem Kollagenhydrolysat und ein Präbiotikum im Gewichtsverhältnis 1:1 gemischt wurden.

Als Kollagenhydrolysat wurde das Produkt GELITA® Sol der Anmelderin eingesetzt, bei dem es sich um enzymatisch hydrolysiertes Kollagen aus Rinderspalt mit einem mittleren Molekulargewicht von ca. 3.000 Da handelt.

Als präbiotisches Polysaccharid wurde Inulin verwendet, das unter der Bezeichnung Orafti® Synergy1 von der Fa. BENEO-Orafti vertrieben wird.

Die erfindungsgemäße Zusammensetzung sowie als Vergleich die beiden einzelnen Bestandteile wurden jeweils in einer Menge von 60 g/l in Wasser gelöst und einer sensorischen Beurteilung durch sieben Testpersonen unterzogen. Dabei nahmen die Testpersonen jeweils eine Beurteilung der folgenden fünf Geschmackskomponenten auf einer Skala von 0 (nicht wahrnehmbar) bis 6 (stark ausgeprägt) vor:

| | |
|---|---|
| A: | leimig |
| B: | bitter |
| C: | sauer |
| D: | süß |
| E: | karamellig |

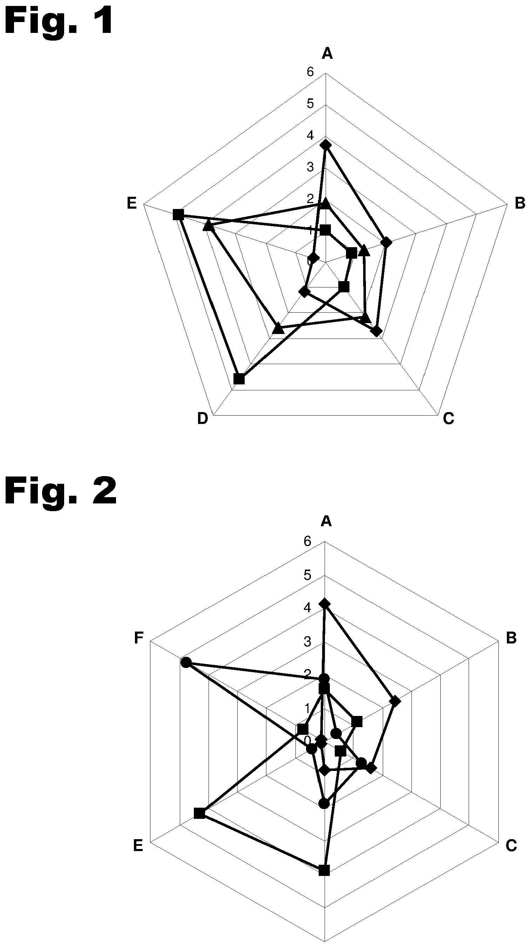

Aus den Einzelbewertungen der sieben Testpersonen wurden jeweils die Mittelwerte gebildet und in einem Netzdiagramm mit fünf sternförmig angeordneten Achsen (für die Geschmackskomponenten A bis E) dargestellt. Dieses Netzdiagramm ist in der Figur 1 gezeigt, wobei die Werte für die erfindungsgemäße Zusammensetzung (Kollagenhydrolysat mit Inulin) als Quadrate, die Werte für das Kollagenhydrolysat als Rauten und die Werte für das Inulin als Dreiecke dargestellt sind.

Es zeigt sich deutlich, dass beim Kollagenhydrolysat die typischerweise als unangenehm empfundenen Geschmackskomponenten (leimig, bitter und sauer) dominieren. Dem gegenüber wird der Geschmack des Inulins im Wesentlichen als süß und karamellig beurteilt.

Überraschenderweise wird der geschmackliche Eindruck der erfindungsgemäßen Zusammensetzung im Durchschnitt noch positiver beurteilt als das Inulin alleine, d.h. als weniger leimig, bitter und sauer sowie stärker karamellig und wesentlich süßer. Der unangenehme Geschmack des Kollagenhydrolysats wird durch die Mischung von Inulin also nicht nur abgemildert, sondern ins Gegenteil verkehrt.

Durch diesen geschmacklichen Effekt kann die Akzeptanz der erfindungsgemäßen Zusammensetzung beim Anwender gegenüber reinem Kollagenhydrolysat deutlich verbessert werden, was neben der synergetischen Wirkung im Hinblick auf die Gesundheit der Knochen einen weiteren wesentlichen Vorteil darstellt.

### Beispiel 2

Es wurde eine weitere erfindungsgemäße Zusammensetzung mit einem anderen Präbiotikum hergestellt, indem Kollagenhydrolysat (GELITA® Sol) im Gewichtsverhältnis 1:1 mit einem Produkt, das einen hohen Anteil an Galacto-Oligosacchariden enthält, gemischt wurde (VIVINAL®, vertrieben von der Fa. Friesland Campina Domo).

Es wurde wiederum eine sensorische Beurteilung durch sieben Testpersonen durchgeführt, und zwar für die in Beispiel 1 beschriebene Zusammensetzung (Kollagenhydrolysat mit Inulin), die Mischung aus Kollagenhydrolysat und Galacto-Oligosacchariden sowie Kollagenhydrolysat alleine.

Es wurden die Geschmackskomponenten A bis E gemäß dem Beispiel 1 beurteilt sowie zusätzlich die Geschmackskomponente "milchig", wiederum auf einer Skala von 0 bis 6.

Die Ergebnisse sind in dem in der Figur 2 gezeigten Netzdiagramm dargestellt. Die Symbole für Kollagenhydrolysat (Rauten) und Kollagenhydrolysat mit Inulin (Quadrate) entsprechen der Figur 1, die Werte für Kollagenhydrolysat mit Galacto-Oligosacchariden sind durch Kreise dargestellt.

Auch im Fall dieser zweiten erfindungsgemäßen Zusammensetzung zeigt sich eine deutliche Verschiebung des Geschmackseindrucks gegenüber dem Kollagenhydrolysat, insbesondere die Geschmackskomponenten "leimig" und "bitter" sind deutlich reduziert. Im Gegensatz zu der ersten Zusammensetzung wird die Mischung aus Kollagenhydrolysat und Galacto-Oligosacchariden im Wesentlichen als "milchig" beschrieben, wobei es sich aber ebenfalls um eine als angenehm empfundene Geschmacksrichtung handelt.

## Patentansprüche

1. Zusammensetzung für Ernährungszwecke für die Verwendung zur Vorbeugung und/oder Behandlung von Osteoporose, umfassend 20 bis 80 Gew.% Kollagenhydrolysat mit einem mittleren Molekulargewicht im Bereich von 1500 bis 8000 Da und 20 bis 80 Gew.% eines oder mehrerer Präbiotika, jeweils bezogen auf die Trockenmasse der Zusammensetzung.

2. Zusammensetzung für die Verwendung nach Anspruch 1, wobei der Anteil des Kollagenhydrolysats und des oder der Präbiotika jeweils 40 bis 60 Gew.% beträgt, jeweils bezogen auf die Trockenmasse der Zusammensetzung.

3. Zusammensetzung für die Verwendung nach Anspruch 1 oder 2, wobei das Kollagenhydrolysat durch enzymatische Hydrolyse von Kollagen aus tierischem Bindegewebe oder Knochen, insbesondere von Schweinen, Rindern, Geflügel oder Fischen, hergestellt ist.

4. Zusammensetzung für die Verwendung nach einem der vorangehenden Ansprüche, wobei das Kollagenhydrolysat ein mittleres Molekulargewicht im Bereich von 2500 bis 3500 Da aufweist.

5. Zusammensetzung für die Verwendung nach einem der vorangehenden Ansprüche, wobei das oder die Präbiotika ausgewählt sind aus Oligo- und/oder Polysacchariden, insbesondere aus Inulin, Fruktanen, Galacto-Oligosacchariden (GOS), Fructo-Oligosacchariden (FOS), resistenten Maltodextrinen, Polydextrose und Mischungen hiervon.

6. Zusammensetzung für die Verwendung nach einem der vorangehenden Ansprüche, ferner umfassend mindestens ein lösliches Calciumsalz.

7. Zusammensetzung für die Verwendung nach Anspruch 6, wobei das mindestens eine lösliche Calciumsalz ausgewählt ist aus Calciumcitrat, Calciumlactat, Calcium-gluconat, Calciumlactatgluconat, Calciumlactobionat und Mischungen hiervon.

8. Zusammensetzung für die Verwendung nach Anspruch 6 oder 7, wobei der Anteil an Calcium 0.1 bis 10 Gew.% beträgt, bevorzugt 0.5 bis 5 Gew.%, bezogen auf die Trockenmasse der Zusammensetzung.

9. Zusammensetzung für die Verwendung nach einem der vorangehenden Ansprüche, ferner umfassend Vitamin D, Vitamin K, Vitamin C, Mineralstoffe und/oder Glucosamin.

10. Zusammensetzung für die Verwendung nach einem der vorangehenden Ansprüche, wobei die Zusammensetzung in Form eines Pulvers oder Granulats vorliegt.

11. Zusammensetzung für die Verwendung nach einem der Ansprüche 1 bis 9, wobei die Zusammensetzung in Form einer Lösung oder einer Suspension vorliegt.

12. Zusammensetzung für die Verwendung nach einem der vorangehenden Ansprüche als Nahrungsergänzungsmittel, insbesondere in Form von Tabletten, Kapseln, Capsetten oder Sachets.

13. Zusammensetzung für die Verwendung nach Anspruch 12, wobei eine Tagesdosis 3 bis 15 g der erfindungsgemäßen Zusammensetzung enthält, bevorzugt 5 bis 10 g.

14. Zusammensetzung für die Verwendung nach einem der Ansprüche 1 bis 11 als Bestandteil von Lebensmitteln, insbesondere Milchprodukten oder Süßwaren.

## Claims

1. Composition for nutritional purposes for preventing and/or treating osteoporosis, comprising 20 to 80 % by weight of collagen hydrolysate with an average molecular weight in the range from 1,500 to 8,000 Da and 20 to 80 % by weight of one or more prebiotics, in each case based on the dry mass of the composition.

2. Composition for the use according to claim 1, wherein the proportion of the collagen hydrolysate and of the prebiotic(s) is in each case 40 to 60 % by weight, in each case based on the dry mass of the composition.

3. Composition for the use according to claim 1 or 2, wherein the collagen hydrolysate is produced by enzymatic hydrolysis of collagen from animal connective tissue or bone, in particular from pigs, cattle, poultry or fish.

4. Composition for the use according to any one of the preceding claims, wherein the collagen hydrolysate has an average molecular weight in the range from 2,500 to 3,500 Da.

5. Composition for the use according to any one of the preceding claims, wherein the prebiotic(s) are selected from oligosaccharides and/or polysaccharides, in particular from inulin, fructans, galacto-oligosaccharides (GOS), fructo-oligosaccharides (FOS), resistant maltodextrins, polydextrose and mixtures thereof.

6. Composition for the use according to any one of the preceding claims, further comprising at least one soluble calcium salt.

7. Composition for the use according to claim 6, wherein the at least one soluble calcium salt is selected from calcium citrate, calcium lactate, calcium gluconate, calcium lactate gluconate, calcium lactobionate and mixtures thereof.

8. Composition for the use according to claim 6 or 7, wherein the proportion of calcium is 0.1 to 10 % by weight, preferably 0.5 to 5 % by weight, based on the dry mass of the composition.

9. Composition for the use according to any one of the preceding claims, further comprising vitamin D, vitamin K, vitamin C, minerals and/or glucosamine.

10. Composition for the use according to any one of the preceding claims, wherein the composition is in the form of a powder or granulate.

11. Composition for the use according to any one of claims 1 to 9, wherein the composition is in the form of a solution or a suspension.

12. Composition for the use according to any one of the preceding claims as a food supplement, in particular in the form of tablets, capsules, capsettes or sachets.

13. Composition for the use according to claim 12, wherein a daily dose contains 3 to 15 g of the composition according to the invention, preferably 5 to 10 g.

14. Composition for the use according to any one of claims 1 to 11 as a constituent of food, in particular milk products or confectionery.

## Revendications

1. Composition à des fins nutritives destinée à être utilisée dans la prévention et/ou le traitement de l'ostéoporose, comprenant 20 à 80 % en poids d'hydrolysat de collagène ayant un poids moléculaire moyen dans la plage de 1500 à 8000 Da et 20 à 80 % en poids d'un ou de plusieurs prébiotiques, respectivement par rapport à la matière sèche de la composition.

2. Composition destinée à être utilisée selon la revendication 1, dans laquelle la proportion d'hydrolysat de collagène et du ou des prébiotiques est respectivement de 40 à 60 % en poids, respectivement par rapport à la matière sèche de la composition.

3. Composition destinée à être utilisée selon la revendication 1 ou 2, dans laquelle l'hydrolysat de collagène est fabriqué par hydrolyse enzymatique du collagène à partir de tissu conjonctif ou d'os animal, en particulier de porcs, de bovins, de volailles ou de poissons.

4. Composition destinée à être utilisée selon l'une des revendications précédentes, dans laquelle l'hydrolysat de collagène présente un poids moléculaire moyen dans la plage de 2500 à 3500 Da.

5. Composition destinée à être utilisée selon l'une des revendications précédentes, dans laquelle le ou les prébiotiques sont choisis parmi les oligo- et/ou polysaccharides, en particulier l'inuline, les fructanes, les galacto-oligosaccharides (GOS), les fructo-oligosaccharides (FOS), les maltodextrines résistantes, la polydextrose et des mélanges de ceux-ci.

6. Composition destinée à être utilisée selon l'une des revendications précédentes, comprenant en outre au moins un sel de calcium soluble.

7. Composition destinée à être utilisée selon la revendication 6, dans laquelle au moins un sel de calcium soluble est choisi parmi le citrate de calcium, le lactate de calcium, le gluconate de calcium, le gluconate-lactate de calcium, le lactobionate de calcium et des mélanges de ceux-ci.

8. Composition destinée à être utilisée selon la revendication 6 ou 7, dans laquelle la proportion de calcium est de 0,1 à 10 % en poids, de préférence 0,5 à 5 % en poids par rapport à la matière sèche de la composition.

9. Composition destinée à être utilisée selon l'une des revendications précédentes, comprenant en outre de la vitamine D, de la vitamine K, de la vitamine C, des substances minérales et/ou de la glucosamine.

10. Composition destinée à être utilisée selon l'une des revendications précédentes, dans laquelle la composition se présente sous la forme d'une poudre ou d'un granulé.

11. Composition destinée à être utilisée selon l'une des revendications 1 à 9, dans laquelle la composition se présente sous la forme d'une solution ou d'une suspension.

12. Composition destinée à être utilisée selon l'une des revendications précédentes à titre de complément alimentaire, en particulier sous la forme de comprimés, gélules, capsules, ou sachets.

13. Composition destinée à être utilisée selon la revendication 12, dans laquelle une dose quotidienne comprend de 3 à 15 g de la composition selon l'invention, de préférence de 5 à 10 g.

14. Composition destinée à être utilisée selon l'une des revendications 1 à 11 en tant que composant de produits alimentaires, en particulier de produits laitiers ou de confiseries.
